# EUROPEAN PATENT SPECIFICATION

(11) **EP 3 930 611 B1**
(45) Date of publication and mention of the grant of the patent: **28.10.2026**
(21) Application number: 20714762.0
(22) Date of filing: 26.02.2020
(51) Int. Cl.: A61B 18/12, A61B 18/00

(54) **MODULAR DOCKING SYSTEM FOR ELECTROSURGICAL EQUIPMENT**
MODULARES ANDOCKSYSTEM FÜR ELEKTROCHIRURGISCHE AUSRÜSTUNG
SYSTÈME D'ACCUEIL MODULAIRE POUR ÉQUIPEMENT ÉLECTROCHIRURGICAL

(30) Priority: 26.02.2019 US 201962810486 P
(43) Date of publication of application: 05.01.2022
(73) Proprietor: CONMED Corporation, Largo, FL 33773 (US)
(72) Inventor: FRAME, Dan, Denver, CO 80206 (US); COOK, John, Conifer, CO 80433 (US)
(74) Representative: Strehl & Partner mbB
(86) International application number: PCT/US2020/019821
(87) International publication number: WO 2020/176578

(56) References cited:
- US-A- 5 601 445
- US-A1- 2004 177 380
- US-A1- 2010 069 939
- US-A1- 2016 296 271
- US-A1- 2017 095 286
- US-A1- 2017 202 594
- US-A1- 2018 238 776

## Description

### BACKGROUND OF THE INVENTION

### 1. FIELD OF THE INVENTION

The present invention relates to surgical systems and, more specifically, to a system for interconnecting electrosurgical systems to reduce complexity and optimize space.

### 2. DESCRIPTION OF THE RELATED ART

While the addition of innovative and novel technology is needed and desired within the operating room, logistical issues, such as space and power constraints, often arise and can inhibit the introduction of new technology. For example, new modules or devices can take up valuable space within the operating room. In addition, operating rooms may also have a limited number of power outlets in a limited number of locations, resulting in a user having to place devices in non-ideal locations, which can become a safety hazard.

As an example, smoke evacuators used in combination with electrosurgical systems and are typically large modules that require a dedicated power connection. Additionally, smoke evacuators must be positioned relatively close to the electrosurgical unit (ESU) so that the devices can communicate with each other through communication area network (CAN) cables. As a result, there is a need in the field for an approach that allows for multiple systems to be interconnected for power, grounding and communication, thereby reducing complexity and minimizing logistical issues.

US 2010/069 939 A1 discloses an operation system using a male connector and a female connector for coupling medical ultrasonic equipment. US 5 601 445 A discloses structural and electrical connectors for coupling and engaging device modules in a side-by-side arrangement. Further related device connection systems are disclosed in US 2017/095 286 A1, US 2018/238 776 A1, US 2004/177 380 A1, US2017/202 594 A1, and US 2016/296 271 A.

### BRIEF SUMMARY OF THE INVENTION

The present invention provides for a reduced footprint and less cabling in an operating room using the docking system defined in Claim 1. Further advantageous features are set out in the dependent claims.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a perspective view of a modular interconnection system for electrosurgical equipment according to the present invention;
FIG. 2 is a perspective view of a modular docking interface for a smoke evacuator according to the present invention;
FIG. 3 is a perspective of electrical interconnectors in modular docking interface for a smoke evacuator according to the present invention;
FIG. 4 is a perspective view of a modular docking interface for an electrosurgical unit according to the present invention; and
FIG. 5 is a perspective of electrical interconnectors in modular docking interface for an electrosurgical unit according to the present invention.

### DETAILED DESCRIPTION OF THE INVENTION

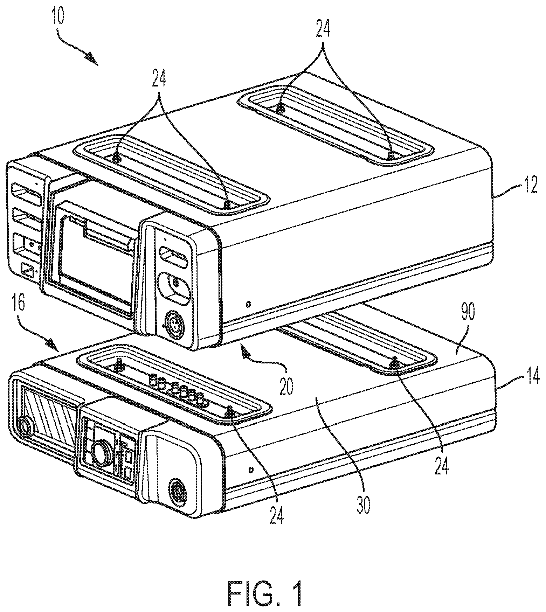

Referring to the figures, wherein like numeral refer to like parts throughout, there is seen in FIG. 1 an electrosurgical system 10 having two or more interconnected modules, shown as an electrosurgical unit 12 and a smoke evacuator 14. Electrosurgical unit 12 and smoke evacuator 14 are interconnected using an upwardly facing docking interface 16 of smoke evacuator 14 that mates with a corresponding downwardly docking interface 20 of electrosurgical unit 12.

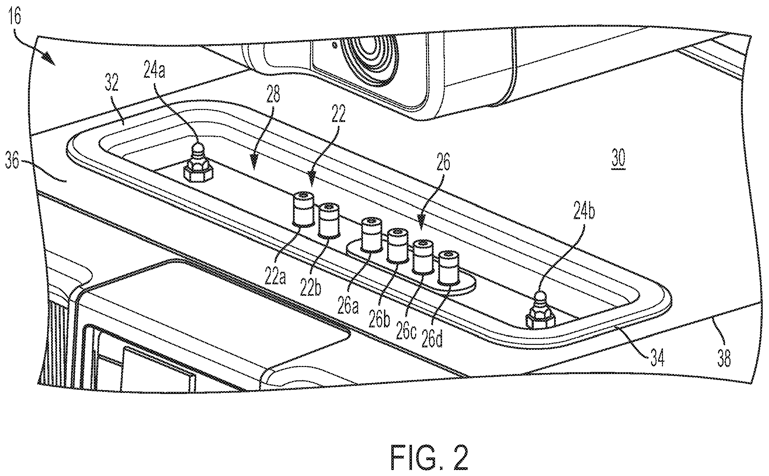

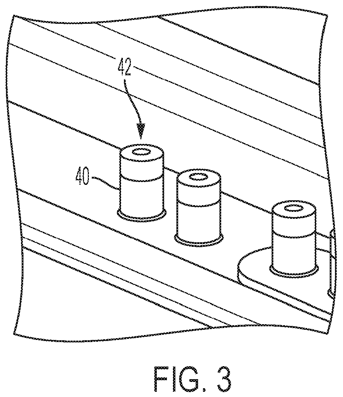

Referring to FIG. 2, docking interface 16 includes a series of power connectors 22, ground studs 24, and communication ports 26, shown as male style connectors positioned within a recessed portion 28 of the housing 30 of smoke evacuator 14. More specifically, docking interface 16 comprises a pair of ground studs 24a and 24b positioned at opposing ends 32 and 34 of recessed portion 28. Recessed portion 28 extends transversely across a forward portion 36 of the upper surface 38 of housing 30. Ground studs 24a and 24b may comprise conventional banana jack plugs that provide electrical connectivity as well as structural coupling. A pair of power connectors 22a and 22b and a series of communication ports 26a, 26b, 26c, and 26d, are positioned and extend between ground studs 24 and 24a and within recessed portion 28. Referring to FIG. 3, power connectors 22a and 22b and a series of communication ports 26a, 26b, 26c, and 26d may comprise cylindrical posts 40 that extend outwardly and upwardly from housing 30, wherein each post 40 defines an inner cavity 42 having electrical contacting faces therein as is conventionally known for electronic interconnection.

Power connectors 22a and 22b, ground studs 24 and 24a, and communication ports 26a, 26b, 26c, and 26d are arranged linearly and extend transversely across forward portion 36 of housing 30. This orientation provides structural stability to the interconnection between electrosurgical unit 12 and smoke evacuator 14 as well as electrical connectivity when electrosurgical unit 12 and smoke evacuator 14 are interconnected. The orientation also allows for easy interconnection in the field and ensure that electrosurgical unit 12 and smoke evacuator 14 are aligned in a condensed footprint to minimize the amount of space needed for electrosurgical unit 12 and smoke evacuator 14.

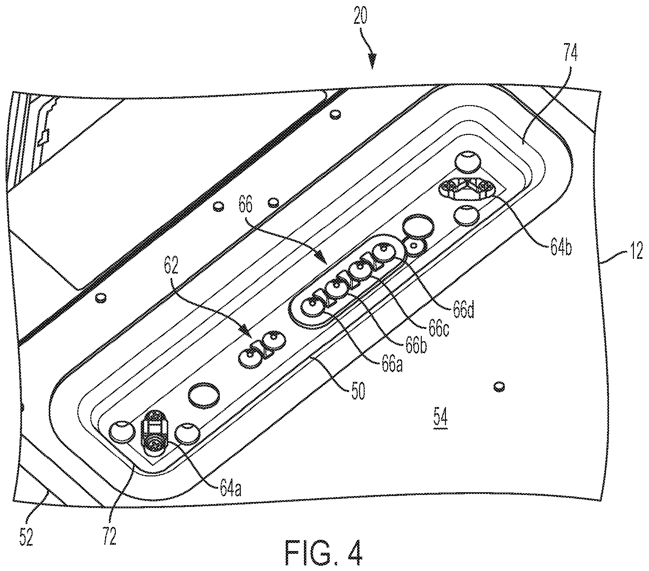

Referring to FIG. 4, docking interface 20 of electrosurgical unit 12 comprises a raised portion 50 relative to a housing 54 of electrosurgical unit 12. Raised portion 50 extends downwardly from a forward portion 52 of the lower surface of housing 54. The geometry of docking interface 20 corresponds to the geometry of docking interface 16 so that docking interface 20 may be received stably within docking interface 16. For example, docking interface 16 is shown as generally rectangular with a slight taper and radius defining the perimeter of docking interface 16. Docking interface 20 has a corresponding rectangular shape with a slight taper and radius so that docking interface 16 and docking interface 20 can be easily positioned by a user in an interfacing relationship and so that when docking interface 20 is seated with docking interface 16, there is contact between the rectangular surfaces to provide structural stability between electrosurgical unit 12 and a smoke evacuator 14 as they are stacked together.

Docking interface 20 includes series of power connectors 62, ground stud receivers 64a and 64b, and communication ports 46, shown in FIG. 4 as female connectors positioned within raised portion 50 of housing 54 of electrosurgical unit 12. In the example of banana jack plugs as ground studs 24a and 24b, ground stud receivers 64a and 64b may comprise banana jack sockets. As with docking interface 26, docking interface 20 includes a pair of ground stud receivers 64a and 64b positioned at opposing ends 72 and 74 of raised portion 50.

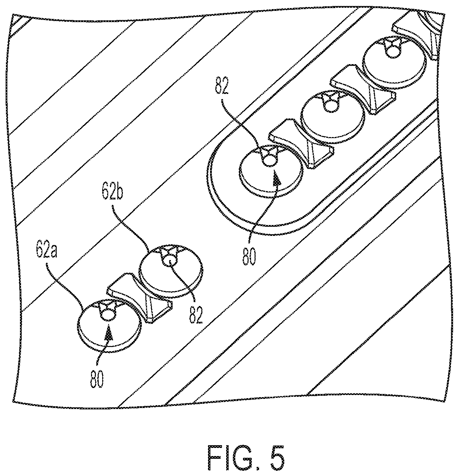

As seen in FIG. 5, power connectors 62a and 62b and a series of communication ports 66a, 66b, 66c, and 66d may comprise cylindrical receptacles 80 extending inwardly into housing 54 and having a contact pins 82 supported therein for electrical engagement with inner cavities 42 of power connectors 22a and 22b and communication ports 26a, 26b, 26c, and 26d.

Power connectors 62, ground connectors 64, and communication ports 66 and positioned to engage power connectors 22, ground studs 24, and communication connectors 26 and provide for electrical continuity therebetween when smoke evacuator 14 is positioned on electrosurgical unit 12 so that docking interface 20 is received within docking interface 16. As a result, smoke evacuator 14 can be powered from electrosurgical unit 12 and be placed into electrical communication therewith without the need for an external power cable or a communication interface cable. Instead, simply positioning smoke evacuator 14 on top of electrosurgical unit 12 with provide for power and communication interconnectivity. The connection ports and grounding studs also act to hold the modules together, so they do not come apart unless the user wants to take them apart. The interfacing of power connectors 62, ground connectors 64, and communication ports 66 with power connectors 22, ground stud receivers 24, and communication ports 26 also helps hold the modules together.

As seen in FIG. 1, smoke evacuator 14 may include an additional receptacle 16 positioned in rear portion 90 that includes additional ground contacts 24. Similarly, electrosurgical unit 12 may include an additional docking interface 20 containing just ground connectors 24 positioned on a rear portion of the downwardly surface. As further seen in FIG. 1, additional upwardly facing docking interfaces 16 may be provided on an upper surface of electrosurgical unit 12 for interconnection to other operating room equipment outfitting with docking interfaces according to the present invention. It should be recognized that the docking interfaces of the present invention could be reversed, *i.e*., the recessed and raised portions reverses, or the positioning of the electrosurgical unit 12 and smoke evacuator 14 reversed with the docking interfaces reverses accordingly.

## Claims

1. A docking system for electrosurgical equipment, comprising:
a first electrosurgical device (14) having a first housing (30); and
a second electrosurgical device (12) having a second housing (54); wherein
the first electrosurgical device comprises a first docking interface (16) comprising a recessed portion (28) in the first housing (30) of the first electrosurgical device and extending transversely across a forward portion of the upper surface of the first housing, wherein the recessed portion includes a first set of electrical contacts (22, 26) arranged linearly and extending transversely thereacross;
the second electrosurgical device comprises a second docking interface (20) comprising a raised portion (50) in the second housing (54) of the second electrosurgical device and extending transversely across a forward portion of the lower surface of the second housing, wherein the raised portion includes a second set of electrical contacts (62, 66) arranged linearly and extending transversely thereacross; and
the recess is configured to receive the raised portion therein so that the first set of electrical contacts are coupled to and in electrical communication with the second set of electrical contacts when the first docking interface and second docking interface are positioned together.

2. The docking system of claim 1, wherein the first electrosurgical device comprises a smoke evacuator (14).

3. The docking system of claim 2, wherein the first set of electrical contacts (22, 26) are positioned on an upper surface the smoke evacuator (14).

4. The docking system of claim 3, wherein the second electrosurgical device comprises an electrosurgical unit (12).

5. The docking system of claim 4, wherein the second set of electrical contacts (62, 66) are positioned on a lower surface of the electrosurgical unit (12).

6. The docking system of claim 5, wherein the first set of electrical contacts and the second set of electrical contacts each comprise a pair of ground contacts (24, 64), a pair of power contacts (22, 62), and a series of communication contacts (26, 66).

7. The docking system of claim 6, wherein the smoke evacuator (14) is adapted to receive power from the electrosurgical unit (12) via the first docking interface (16) and the second docking interface (20).

8. The docking system of claim 7, wherein the smoke evacuator (14) is in communication with the electrosurgical unit (12) via the first docking interface (16) and the second docking interface (20).

9. The docking system of claim 8, wherein the first docking interface (16) extends transversely across an upper surface of the first housing (30) of the smoke evacuator (14).

10. The docking system of claim 9, wherein the second docking interface (20) extends transversely across a lower surface of the second housing (54) of the electrosurgical unit (12).

11. The docking system of claim 10, wherein the upper surface of the first housing (30) of the smoke evacuator (14) includes a third docking interface (16) comprising a recess.

12. The docking system of claim 11, wherein the lower surface of the second housing (54) of the electrosurgical unit (12) includes a fourth docking interface (20) comprising a raised portion that can be received by the recess of the third docking interface (16).

13. The docking system of claim 12, wherein the first docking interface (16) extends across a front portion of the smoke evacuator (12), the third docking interface (16) extends across a rear portion (90) of the smoke evacuator, the second docking interface (20) extends across a front portion of the electrosurgical unit (14), and the fourth docking interface (20) extends across a rear portion of the electrosurgical unit.

## Patentansprüche

1. Andocksystem für elektrochirurgische Ausrüstung, umfassend:
ein erstes elektrochirurgisches Gerät (14) mit einem ersten Gehäuse (30); und
ein zweites elektrochirurgisches Gerät (12) mit einem zweiten Gehäuse (54); wobei
das erste elektrochirurgische Gerät eine erste Andockschnittstelle (16) umfasst, die einen vertieften Abschnitt (28) im ersten Gehäuse (30) des ersten elektrochirurgischen Geräts umfasst und sich quer über einen vorderen Abschnitt der oberen Fläche des ersten Gehäuses erstreckt, wobei der vertiefte Abschnitt einen ersten Satz elektrischer Kontakte (22, 26) aufweist, die linear angeordnet sind und sich quer darüber erstrecken;
das zweite elektrochirurgische Gerät eine zweite Andockschnittstelle (20) umfasst, die einen erhöhten Abschnitt (50) im zweiten Gehäuse (54) des zweiten elektrochirurgischen Geräts umfasst und sich quer über einen vorderen Abschnitt der unteren Fläche des zweiten Gehäuses erstreckt, wobei der erhöhte Abschnitt einen zweiten Satz elektrischer Kontakte (62, 66) aufweist, die linear angeordnet sind und sich quer darüber erstrecken; und
die Vertiefung dazu eingerichtet ist, den erhöhten Abschnitt darin aufzunehmen, so dass der erste Satz elektrischer Kontakte mit dem zweiten Satz elektrischer Kontakte gekoppelt und in elektrischer Verbindung steht, wenn die erste Andockschnittstelle und die zweite Andockschnittstelle zusammenpositioniert sind.

2. Andocksystem nach Anspruch 1, wobei das erste elektrochirurgische Gerät einen Rauchevakuator (14) umfasst.

3. Andocksystem nach Anspruch 2, wobei der erste Satz elektrischer Kontakte (22, 26) auf einer oberen Fläche des Rauchevakuators (14) positioniert ist.

4. Andocksystem nach Anspruch 3, wobei das zweite elektrochirurgische Gerät eine elektrochirurgische Einheit (12) umfasst.

5. Andocksystem nach Anspruch 4, wobei der zweite Satz elektrischer Kontakte (62, 66) auf einer unteren Fläche der elektrochirurgischen Einheit (12) positioniert ist.

6. Andocksystem nach Anspruch 5, wobei der erste Satz elektrischer Kontakte und der zweite Satz elektrischer Kontakte jeweils ein Paar Massekontakte (24, 64), ein Paar Stromkontakte (22, 62) und eine Reihe von Kommunikationskontakten (26, 66) umfassen.

7. Andocksystem nach Anspruch 6, wobei der Rauchevakuator (14) dazu ausgelegt ist, Strom von der elektrochirurgischen Einheit (12) über die erste Andockschnittstelle (16) und die zweite Andockschnittstelle (20) zu empfangen.

8. Andocksystem nach Anspruch 7, wobei der Rauchevakuator (14) über die erste Andockschnittstelle (16) und die zweite Andockschnittstelle (20) in Kommunikation mit der elektrochirurgischen Einheit (12) steht.

9. Andocksystem nach Anspruch 8, wobei sich die erste Andockschnittstelle (16) quer über eine obere Fläche des ersten Gehäuses (30) des Rauchevakuators (14) erstreckt.

10. Andocksystem nach Anspruch 9, wobei sich die zweite Andockschnittstelle (20) quer über eine untere Fläche des zweiten Gehäuses (54) der elektrochirurgischen Einheit (12) erstreckt.

11. Andocksystem nach Anspruch 10, wobei die obere Fläche des ersten Gehäuses (30) des Rauchevakuators (14) eine dritte Andockschnittstelle (16) aufweist, die eine Vertiefung umfasst.

12. Andocksystem nach Anspruch 11, wobei die untere Fläche des zweiten Gehäuses (54) der elektrochirurgischen Einheit (12) eine vierte Andockschnittstelle (20) aufweist, die einen erhöhten Abschnitt umfasst, der von der Vertiefung der dritten Andockschnittstelle (16) aufgenommen werden kann.

13. Andocksystem nach Anspruch 12, wobei sich die erste Andockschnittstelle (16) über einen vorderen Abschnitt des Rauchevakuators (12) erstreckt, sich die dritte Andockschnittstelle (16) über einen hinteren Abschnitt (90) des Rauchevakuators erstreckt, sich die zweite Andockschnittstelle (20) über einen vorderen Abschnitt der elektrochirurgischen Einheit (14) erstreckt und sich die vierte Andockschnittstelle (20) über einen hinteren Abschnitt der elektrochirurgischen Einheit erstreckt.

## Revendications

1. Système d'accueil pour équipement électrochirurgical, comprenant :
un premier dispositif électrochirurgical (14) ayant un premier boîtier (30) ; et
un deuxième dispositif électrochirurgical (12) ayant un deuxième boîtier (54) ; dans lequel
le premier dispositif électrochirurgical comprend une première interface d'accueil (16) comprenant une partie en renfoncement (28) dans le premier boîtier (30) du premier dispositif électrochirurgical et s'étendant transversalement sur une partie avant de la surface supérieure du premier boîtier, dans lequel la partie en renfoncement inclut un premier ensemble de contacts électriques (22, 26) agencés linéairement et s'étendant transversalement sur celle-ci ;
le deuxième dispositif électrochirurgical comprend une deuxième interface d'accueil (20) comprenant une partie en surélévation (50) dans le deuxième boîtier (54) du deuxième dispositif électrochirurgical et s'étendant transversalement sur une partie avant de la surface inférieure du deuxième boîtier, dans lequel la partie en surélévation inclut un deuxième ensemble de contacts électriques (62, 66) agencés linéairement et s'étendant transversalement sur celle-ci ; et
le renfoncement est configuré pour recevoir la partie en surélévation dans celui-ci de telle sorte que le premier ensemble de contacts électriques sont couplés au et en communication électrique avec le deuxième ensemble de contacts électriques lorsque la première interface d'accueil et la deuxième interface d'accueil sont positionnées ensemble.

2. Système d'accueil selon la revendication 1, dans lequel le premier dispositif électrochirurgical comprend un évacuateur (14) de fumées.

3. Système d'accueil selon la revendication 2, dans lequel le premier ensemble de contacts électriques (22, 26) sont positionnés sur une surface supérieure de l'évacuateur (14) de fumées.

4. Système d'accueil selon la revendication 3, dans lequel le deuxième dispositif électrochirurgical comprend une unité électrochirurgicale (12).

5. Système d'accueil selon la revendication 4, dans lequel le deuxième ensemble de contacts électriques (62, 66) sont positionnés sur une surface inférieure de l'unité électrochirurgicale (12).

6. Système d'accueil selon la revendication 5, dans lequel le premier ensemble de contacts électriques et le deuxième ensemble de contacts électriques comprennent chacun une paire de contacts de masse (24, 64), une paire de contacts d'alimentation électrique (22, 62), et une série de contacts de communication (26, 66).

7. Système d'accueil selon la revendication 6, dans lequel l'évacuateur (14) de fumées est adapté à recevoir une alimentation électrique depuis l'unité électrochirurgicale (12) par l'intermédiaire de la première interface d'accueil (16) et de la deuxième interface d'accueil (20).

8. Système d'accueil selon la revendication 7, dans lequel l'évacuateur (14) de fumées est en communication avec l'unité électrochirurgicale (12) par l'intermédiaire de la première interface d'accueil (16) et de la deuxième interface d'accueil (20).

9. Système d'accueil selon la revendication 8, dans lequel la première interface d'accueil (16) s'étend transversalement sur une surface supérieure du premier boîtier (30) de l'évacuateur (14) de fumées.

10. Système d'accueil selon la revendication 9, dans lequel la deuxième interface d'accueil (20) s'étend transversalement sur une surface inférieure du deuxième boîtier (54) de l'unité électrochirurgicale (12).

11. Système d'accueil selon la revendication 10, dans lequel la surface supérieure du premier boîtier (30) de l'évacuateur (14) de fumées inclut une troisième interface d'accueil (16) comprenant un renfoncement.

12. Système d'accueil selon la revendication 11, dans lequel la surface inférieure du deuxième boîtier (54) de l'unité électrochirurgicale (12) inclut une quatrième interface d'accueil (20) comprenant une partie en surélévation qui peut être reçue par le renfoncement de la troisième interface d'accueil (16).

13. Système d'accueil selon la revendication 12, dans lequel la première interface d'accueil (16) s'étend au travers d'une partie avant de l'évacuateur (14) de fumées, la troisième interface d'accueil (16) s'étend au travers d'une partie arrière (90) de l'évacuateur de fumées, la deuxième interface d'accueil (20) s'étend au travers d'une partie avant de l'unité électrochirurgicale (14) et la quatrième interface d'accueil (20) s'étend au travers d'une partie arrière de l'unité électrochirurgicale.
